# EUROPEAN PATENT SPECIFICATION

(11) **EP 2 664 623 B1**
(45) Date of publication and mention of the grant of the patent: **04.03.2015**
(21) Application number: 13180582.2
(22) Date of filing: 09.09.2004
(51) Int. Cl.: C07K 11/02, C07K 7/64, A61K 38/15

(54) **New antitumoral compounds**
Neue Antitumor-Verbindungen
Nouveaux composés antitumoraux

(30) Priority: 09.09.2003 GB 0321066
(43) Date of publication of application: 20.11.2013
(62) Divisional of application: 10179452.7
(73) Proprietor: Pharma Mar, S.A.U., 28770 Madrid (ES)
(72) Inventor: Fernandez Donis, Ariadna, 08028 Barcelona (ES); Giralt Lledó, Ernest, 08028 Barcelona (ES); Gracia Cantador, Carolina, 08028 Barcelona (ES); López Rodríguez, Pilar, 08028 Barcelona (ES); Varon Colomer, Sonia, 08028 Barcelona (ES); Cuevas Marchante, Carmen, 28770 Madrid (ES); López Macia, Ángel, 28770 Madrid (ES); Francesch Solloso, Andrés, 28770 Madrid (ES); Jiménez García, José-Carlos, 08028 Barcelona (ES); Royo Expósito, Miriam, 08028 Barcelona (ES); Albericio Palomera, Fernando, 08028 Barcelona (ES)
(74) Representative: Andrews, Timothy Stephen

(56) References cited:
- WO-A-01/58934
- WO-A-03/033012
- WO-A-2004/035613
- HAMANN M T ET AL: "Kahalalides: bioactive peptides from a marine mollusk Elysia rufescens and its algal diet Bryopsis sp", JOURNAL OF ORGANIC CHEMISTRY, AMERICAN CHEMICAL SOCIETY. EASTON, US, vol. 61, no. 19, 1996, pages 6594-6600, XP002125310, ISSN: 0022-3263
- LÓPEZ-MACIÀ A ET AL: "Synthesis and structure determination of kahalalide F (1,2).", JOURNAL OF THE AMERICAN CHEMICAL SOCIETY. 21 NOV 2001, vol. 123, no. 46, 21 November 2001 (2001-11-21), pages 11398-11401, XP002313084, ISSN: 0002-7863

## Description

### FIELD OF THE INVENTION

The present invention is directed to new kahalalide antitumoral compounds, in particular to analogues of kahalalide F, pharmaceutical compositions containing them and their use as antitumoral, antiviral, antifungal agents and in the treatment of psoriasis.

### BACKGROUND OF THE INVENTION

The kahalalide compounds are peptides isolated from a Hawaiian herbivorous marine species of mollusc, *Elysia rufescens* and its diet, the green alga *Bryopsis sp..* Kahalalide F is described in Hamann, M et al., J. Am. Chem. Soc., 1993, 115, 5825-5826.

Kahalalide A-G are described in Hamann, M. et al., J. Org. Chem, 1996, 61, 6594-6600: "Kahalalides: bioactive peptides from a marine mollusk *Elysia rufescens* and its algal diet *Bryopsis* sp.".

Kahalalide H and J are described in Scheuer P.J. et al., J. Nat. Prod. 1997, 60, 562-567: "Two acyclic kahalalides from the sacoglossan mollusk Elysia rufescens*".*

Kahalalide O is described in Scheuer P.J. et al., J. Nat Prod. 2000, 63(1) 152-4: "A new depsipeptide from the sacoglossan mollusk Elysia ornata and the green alga Bryopsis species".

For kahalalide K, see Kan, Y. et al., J. Nat. Prod. 1999 62(8) 1169-72: "Kahalalide K: A new cyclic depsipeptide from the hawaiian green alga Bryopsis species".

For related reports, see also Goetz et al., Tetrahedron, 1999, 55; 7739-7746: "The absolute stereochemistry of Kahalalide F"; Albericio, F. et al. Tetrahedron Letters, 2000, 41, 9765-9769: "Kahalalide B. Synthesis of a natural cyclodepsipeptide"; Becerro et al. J. Chem. Ecol. 2001, 27(11), 2287-99: "Chemical defenses of the sarcoglossan mollusk Elysia rufescens and its host Alga bryopsis sp.".

Of the kahalalide compounds, kahalalide F is the most promising because of its antitumoral activity. Its structure is complex, comprising six amino acids as a cyclic part, and an exocyclic chain of seven amino acids with a terminal fatty acid group. Originally kahalalide F was reported to have the structure (I).

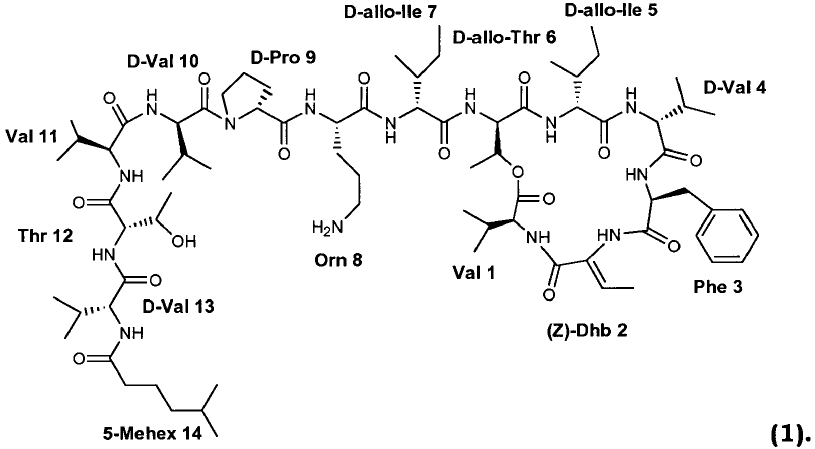

In WO 04035613 there is described the 4(S)-methylhexyl compound with the following formula (II).

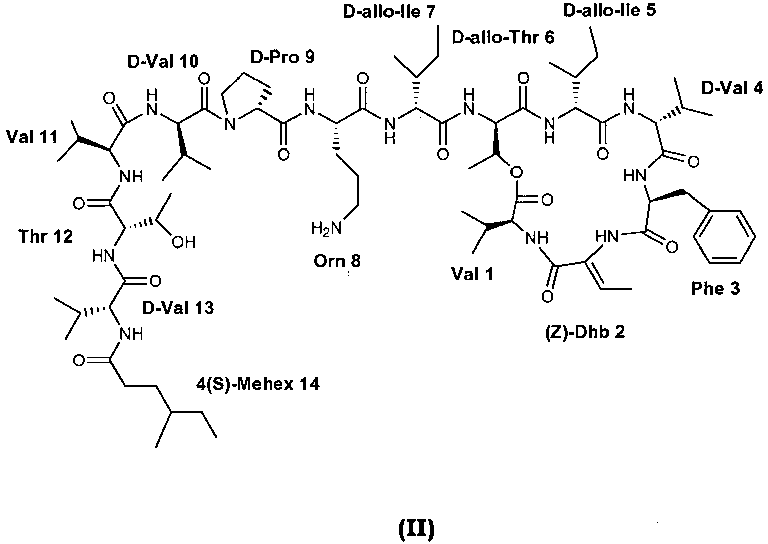

The activity of kahalalide F against *in vitro* cell cultures of human lung carcinoma A-549 and human colon carcinoma HT-29 were reported in EP 610 078. Kahalalide F has also demonstrated to have antiviral and antifungal properties, as well as to be useful in the treatment of psoriasis.

WO 02 36145 describes pharmaceutical compositions containing kahalalide F and new uses of this compound in cancer therapy.

WO 03 33012 describes the clinical use in oncology of kahalalide compounds.

The synthesis and cytotoxic activities of natural and synthetic kahalalide compounds is described in WO 01 58934. WO 01 58934 describes the synthesis of kahalalide F and also of compounds with a similar structure in which the terminal fatty acid chain is replaced by other fatty acids.

There is still a need to provide further antitumoral compounds, in particular further kahalalide compounds with improved properties.

### SUMMARY OF THE INVENTION

We have found kahalalide analogue compounds with improved biological activity.

The present invention is directed to a compound based on the structure of Kahalide F of formula 1, designated KF 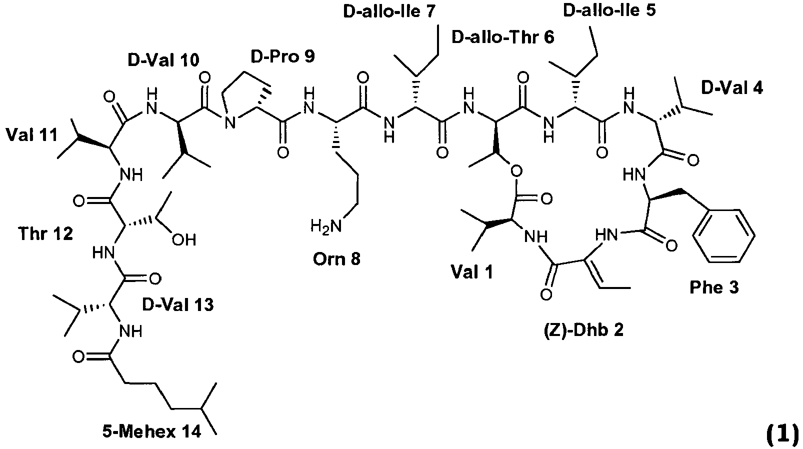 which is [Orn¹¹]-KF, wherein the amino acid indicated between brackets is the modification introduced in the structure of kahalide F; or a pharmaceutically acceptable salt thereof.

The present invention is also directed to a pharmaceutical composition comprising a compound as previously defined and a pharmaceutically acceptable carrier, vehicle or diluent.

In another aspect the present invention is directed to the a compound or composition as defined above for use as a medicament. In a preferred embodiment the compound or composition is for use in the treatment of cancer, psoriasis, viral infection or fungal infection.

The compound of the present invention can be employed for use in the treatment of patients with refractory cancers that do not respond favourably to other treatments. In particular, the compositions of this invention can be employed after other chemotherapy has been tried and not worked.

The present invention is particularly directed to the compounds of the present invention for use in the treatment of patients affected with prostate cancer, breast cancer, hepatocellular carcinoma, melanoma, colorectal cancer, renal cancer, ovarian cancer, NSCL cancer, epithelial cancer, pancreatic cancer and tumors that overexpress the Her2 / neu oncogene.

### DETAILED DESCRIPTION OF THE INVENTION

We have identified an analogue of kahalalide F that shows significant improvement in activity with respect to kahalalide F.

The present invention is directed to a compound based on the structure of Kahalide F of formula 1, designated KF 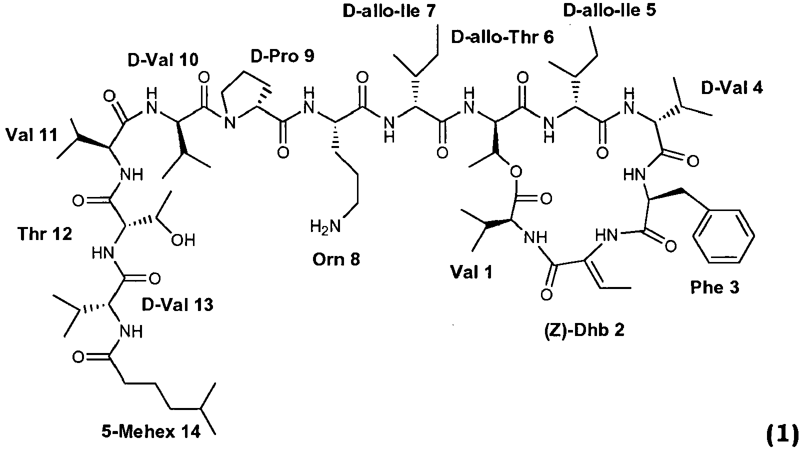 which is [Orn¹¹]-KF, wherein the amino acid indicated between the brackets is the modification introduced into the structure of kahalide F; or a pharmaceutically acceptable salt thereof.

The natural amino acids include alanine, glycine, valine, leucine, isoleucine, phenylalanine, tyrosine, tryptophan, methionine, cysteine, aspartate, asparagine, glutamatic acid, glutamine, lysine, arginine, proline, serine, threonine, histidine and hydroxyproline

We also refer to WO 0158934. That earlier text gives guidance which is applicable to the compound of this invention, and we import the definitions for adoption in the compound of this invention.

Abbreviations used in the following description for amino acids and the designations of peptides follow the rules of the IUPAC IUB Commission of Biochemical Nomenclature in J. Biol. Chem., 1972, 247, 977 983.

| Alloc | allyloxycarbonyl |
|---|---|
| Boc | *tert*-butyloxycarbonyl |
| *t*-Bu | *tert*-Butyl |
| Cl-TrtCl-resin | 2-chlorotrityl chloride-resin |
| Z-Dhb | α,β-didehydro-α-aminobutyric acid |
| DIPEA | N,N-diisopropylethylamine |
| DMF | *N*,*N*-dimethylformamide |
| Fmoc | 9-fluorenylmethoxycarbonyl |
| GI50 | growth inhibition at 50% |
| HOBt | 1-hydroxybenzotriazole |
| LC50 | lethal concentration at 50% |
| MeHex-OH | methyhexanoic acid |
| TFA | trifluoroacetic acid |

Amino acid symbols denote the L-configuration unless stated otherwise.

The present invention also encompass the pharmaceutically acceptable salts, prodrugs, tautomers, and solvates, thereof.

The compound of the present invention has asymmetric centers and therefore exist in different enantiomeric and diastereomic forms. This invention relates to the use of all optical isomers and stereoisomers of the compounds of the present invention, and mixtures thereof, and to all pharmaceutical compositions and methods of treatment that may employ or contain them.

The compound of the invention may be in crystalline form either as a free compound or as a solvate (e.g. a hydrate) and it is intended that both forms are within the scope of the present invention. Methods of solvation are generally known within the art.

The present invention also includes the compound of the present invention, and the pharmaceutically acceptable salts thereof, wherein one or more hydrogen, carbon or other atoms are replaced by isotopes thereof. Such compounds may be useful as research and diagnostic tools in metabolism pharmacokinetic studies and in binding assays.

Salts of the compound of the present invention may comprise acid addition salts derived from a nitrogen in the compound of formula 1 or 2. The therapeutic activity resides in the moiety derived from the compound of the invention as defined herein and the identity of the other component is of less importance although for therapeutic and prophylactic purposes it is, preferably, pharmaceutically acceptable to the patient. Examples of pharmaceutically acceptable acid addition salts include those derived from mineral acids, such as hydrochloric, hydrobromic, phosphoric, metaphosphoric, nitric and sulphuric acids, and organic acids, such as tartaric, acetic, trifluoroacetic, citric, malic, lactic, fumaric, benzoic, glycolic, gluconic, succinic and methanesulphonic and arylsulphonic, for example p-toluenesulphonic, acids. Preferred salts include the trifluoroacetate salt and the hydrochloride salt.

The compound of the present invention can be prepared according to the synthetic process described in WO 01 58934, or according to the improved process as described herein. Therefore also described herein is a process to prepare a compound based on the structure of Kahalide F of formula 1, designated KF, which is [Orn¹¹]-KF, wherein the amino acid indicated between brackets is the modification introduced in the structure of kahalalide F.

The key steps of the optimized process for a more economical and safe synthesis of the analogue of kahalalide F of the invention are: (i) partial incorporation of Fmoc-D-Val-OH onto the chlorotritylchloropolystyrene resin for a initial loading of 0.5 mmol/g; (ii) use as coupling method of DIPCDI-HOBt, instead of HATU-DIPEA, for the sequential incorporation of the protected amino acids and aliphatic carboxylic acids; (iii) cyclization step with DIPCDI/HOBt/DIPEA in CH₂Cl₂; these conditions avoids two side reaction: epimerisation of the Val residue, which is involved in the activation, and trifluoroacetylation of the Phe or its replacement; (iv) use of sodium diethyl-dithiocarbamate after removing Alloc to avoid presence of Pd (0) in the final product.

The synthesis is preferably a solid phase synthetic process.

The preferred embodiment of the synthetic process of the present invention is best represented in the Scheme 1, which is directed to the formation of the target compounds.

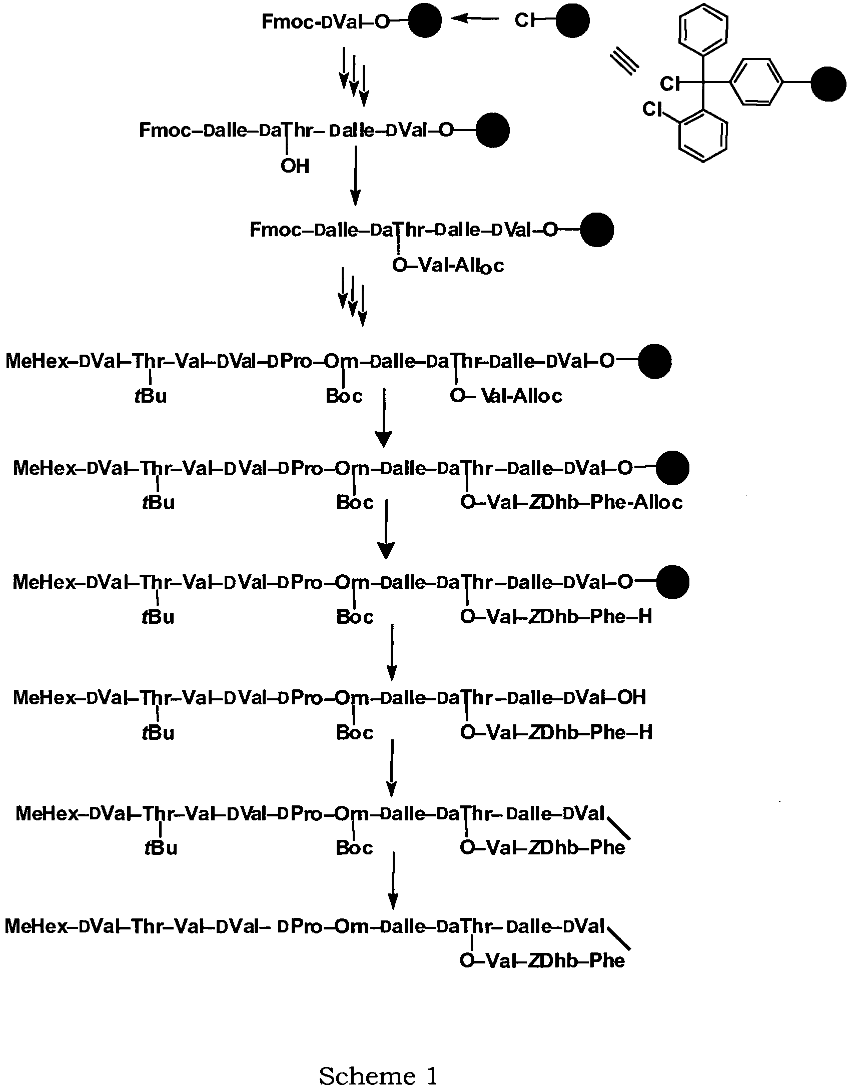

As shown above in Scheme 1, the preferred process for the synthetic formation of analogues of kahalalide F is based in a solid-phase approach, see for example Lloyd-Williams, P., et al. Chemical Approaches to the Synthesis of Peptides and Proteins. CRC Press, Boca Raton (FL), 1997 and followed with modifications of the method already described for the preparation kahalalide F and some of its analogues (WO 01 58934).

The process of Scheme 1 comprises the sequential steps of:
(a) incorporating an Fmoc-DVal-OH onto a chlorotrityl chloro resin, forming an ester bond;
(b) elongating the peptidic chain with three amino acids [DaIle, DaThr (free OH), DaIle) using a Fmoc/ tBu strategy;
(c) incorporating [Val(1)] using an Alloc/tBu strategy;
(d) elongating the peptidic chain with the remaining amino acids and aliphatic carboxylic acids using a Fmoc/*t*Bu strategy;
(e1) incorporating the dipeptide Alloc-Phe-ZDhb-OH, which has been combined and dehydrated in solution;
(e2a) elongating the peptidic chain with two amino acids, preferably Thr and Phe. The OH of Thr is unprotected and the amino group of Phe, or its replacement, is protected with Fmoc or preferably with Alloc; in some cases if it is protected with Fmoc, this is removed and Alloc is introduced in solid-phase;
(e2b) dehydrating in solid-phase to give the didehydropeptide;
(f) removing the Alloc/Fmoc group of Phe, or of its replacement, while the peptide is still anchored to the solid support;
(g) cleaving the side-chain protected peptide from the solid support;
(h) cyclizing the peptide in solution;
(i) removing TFA labile side chain protecting groups.
(j) Further modifications of functional(s) group(s) in solution phase.

Therefore the process can be conducted as follows:
Fmoc-DVal-OH is incorporated preferably to a chlorotritylpolystyrene resin, see Barlos, K.; Gatos, D.; Schäfer, W. Angew. Chem. Int. Ed. Engl. 1991, 30, 590-593, in the presence of DIPEA keeping the level of substitution of aprox. 0.5 mmol/g. The use of higher loadings brings the presence of terminated peptides in the final product, see Chiva, C.; Vilaseca, M.; Giralt, E.; Albericio, F. J. Pept. Sci. 1999, 5, 131-140.

All solvent ratios are volume/volume unless stated otherwise.

Removal of the Fmoc group can be carried out with piperidine-DMF (2:8, v/v) (1 x 2 min, 2 x 10 min). Couplings of Fmoc-aa-OH (4-5 equiv) and the acids at the 14-position can be carried out with DIPCDI-HOBt (equimolar amounts of each one respect to the carboxylic component) or PyBOP-DIPEA (equimolar amount of PyBOP and double amount of DIPEA) in DMF or DMF-Toluene (1:1) for 90 min. After the coupling ninhydrin or chloranil tests are carried out and if it is positive the coupling is repeated in the same conditions, otherwise the process is continued. Washings between deprotection, coupling, and, again, deprotection steps can be carried out with DMF (5 x 0.5 min) and CH₂Cl₂ (5 x 0.5 min) using for example each time 10 mL solvent/g resin.

Incorporation of Alloc-Val-OH (5 equiv) can be carried out with equimolar amount of DIPCDI and 10% of DMAP. This coupling is repeated at least twice.

Removal of Alloc group can be carried out with Pd(PPh₃)₄ (0.1 equiv) in the presence of PhSiH₃ (10 equiv), see Gómez-Martinez, P.; Thieriet, N.; Albericio, F.; Guibé, F. J. Chem. Soc. Perkin I 1999, 2871-2874, and washing the resin with sodium diethyldithiocarbamate in DMF 0.02 M (3 x 15 min).

The dipeptide Alloc-Phe-ZDhb-OH (4 equiv), which was prepared in solution from Alloc-Phe-OH and H-Thr-OtBu with EDC·HCl, and posterior dehydration and treatment with TFA, can be coupled with DIPCDI-HOAt (4 equiv of each) for 5 h to overnight. The use of other coupling reagents based in HOBt, such as HBTU or DIPCDI-HOBt, has led to incomplete incorporations of the dipeptide.

Dehydration can be carried out in solid-phase with EDC·HCl (water soluble carbodiimide, 20 equiv) in the presence of CuCl (12 equiv) in CH₂Cl₂-DMF (9:1) for 7 days. EDC·HCl/CuCl has been used by dehydration in solution of a residue of Thr in a fragment of Nisin (Fukase, K.; Kitazawa, M.; Sano, A.; Shimbo, K.; Horimoto, S.; Fujita, H.; Kubo, A.; Wakamiya, T.; Shibe, A. Bull. Chem. Soc. Jpn. 1992, 65, 2227-2240) and in solid-phase for the preparation of didehydropeptides from Thr, Ser, and phenylserine (Royo, M.; Jiménez, J.C.; López-Macià, A.; Giralt, E.; Albericio, F. Eur. J. Org. Chem. 2001, 45-48).

Cleavage of the protected peptide from the resin can be accomplished by TFA-CH₂Cl₂ (1:99) (5 x 30 sec).

Cyclization step can be carried out with DIPCDI/HOBt/DIPEA in CH₂Cl₂. These conditions avoid two side reactions: epimerisation of the Val residue, which is involved in the activation, and trifluoroacetylation of the Phe or its replacement.

Final deprotection can be carried out with TFA-H₂O (95:5) for 1 hour.

It will be appreciated that the particular choice of protecting groups is not critical, and other choices are widely available. For example, Bzl type groups can replace tBu/Boc; Boc instead of Fmoc; Fmoc instead of Alloc; Wang resin instead of chlorotrityl.

Further detail on the synthesis is given in the examples.

The process of this invention can be carried out from starting materials in an enantio-, stereocontrolled and fast manner, taking advantages of the solid-phase synthetic methodology, where the molecule in construction is bounded to an insoluble support during all synthetic operations.

Pharmaceutical formulations of the compound of the invention may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier (s) or excipient (s).

Preferably pharmaceutical compositions of the compound of the invention include liquid (solutions, suspensions or emulsions) with suitable composition for intravenous administration, and they may contain the pure compound or in combination with any carrier or other pharmacologically active compounds. Further guidance concerning the pharmaceutical compositions can be found in WO 02 361450.

Thus, a combination of a non-ionic surfactant and an organic acid is suited for use with a bulking agent to give a lyophilised form of a compound of the invention suited for reconstitution. Reconstitution is preferably effected with a mix of emulsifying solubiliser, alkanol and water.

The lyophilised composition preferably comprises mainly the bulking agent, such as at least 90 % or at least 95 % bulking agent. Examples of bulking agents are well known and include sucrose and mannitol. Other bulking agents can be employed.

The non-ionic surfactant in the lyophilised composition is preferably a sorbitan ester, more preferably a polyethylene sorbitan ester, such as a polyoxyethylene sorbitan alkanoate, especially a polyoxyethylene sorbitan mono-oleate, for example polysorbate 80. The non-ionic surfactant typically comprises a few % of the composition, such as 0 to 5 % of the composition, for instance 2 to 3 or 4 % of the composition.

The organic acid in the lyophilised composition is typically an aliphatic acid, preferably a hydroxycarboxylic acid and more preferably a hydroxypolycarboxylic acid, notably citric acid. The organic acid typically comprises a few % of the composition, such as 0 to 5 % of the composition, for instance 2 to 3 or 4 % of the composition.

The amount of the compound of the invention in the lyophilised composition is typically less than 1 %, or often less than 0.1 %, of the mix. A suitable amount is in the range 50 to 200 µg, say about 100 µg, per 100 mg of composition.

The emulsifying solubiliser for the reconstituting agent suitably comprises a polyethylene glycol ester, notably an ester of a fatty acid, more preferably a PEG oleate such as PEG-35 oleate. The emulsifying solubiliser is suitably 0 to 10 % of the reconstituting agent, typically about 3 to 7 %, say about 5 %. The alkanol is usually ethanol, and is suitably 0 to 10 % of the reconstituting agent, typically about 3 to 7 %, say about 5 %. The remainder of the reconstituting agent is water, and gives a reconstituted solution suited for intravenous injection.

Further dilution of the reconstituted solution with 0.9 % saline may be appropriate for infusion of the kahalalide compound. Suitable infusion equipment preferably includes a glass container, rather than one of polyethylene. Tubing is preferably of silicone.

The preferred reconstituting agent then comprises 2 to 7 %, say about 5 %, emulsifying solubiliser; 2 to 7 %, say about 5 %, alcohol; and remainder water.

The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use.

Administration of the compound or compositions of the present invention is by intravenous infusion. Infusion times of up to 72 hours can be used, more preferably 1 to 24 hours, with either about 1 or about 3 hours most preferred. Short infusion times which allow treatment to be carried out without an overnight stay in hospital are especially desirable. However, infusion may be around 24 hours or even longer if required.

The administration is performed in cycles, in the preferred application method, an intravenous infusion of a compound of the invention is given to the patients the first week of each cycle, the patients are allowed to recover for the remainder of the cycle. The preferred duration of each cycle is of either 1, 3 or 4 weeks; multiple cycles can be given as needed. In an alternative dosing protocol, the compound of the invention is administered for say about 1 hour for 5 consecutive days every 3 weeks. Other protocols can be devised as variations.

Dose delays and/or dose reductions and schedule adjustments are performed as needed depending on individual patient tolerance of treatments, in particular dose reductions are recommended for patients with higher than normal serum levels of liver transaminases or alkaline phosphatase.

In one aspect, the present invention provides a compound of the invention for use in the treatment of a human patient afflicted with cancer, comprising administering to said patient a compound of the invention at a dose below 1200 mcg/m2/day, preferably below 930 mcg/m2/day and more preferably below 800 mcg/m2/day. Suitably the dose is at least 320 mcg/m2/day. Preferably the dose is in the range of 400-900 mcg/m2/day, preferably 500-800 mcg/m2/day, more preferably 600-750 mcg/m2/day. Especially preferred are doses of about 650-700 mcg/m2/day.

In a further aspect the invention provides a compound of the invention for use in the treatment of a human patient afflicted with cancer, comprising administering to said patient a compound of the invention daily during 5 days at a dose below 930 mcg/m2/day, followed by a resting period of from 1 to 4 weeks in which the kahalalide compound is not administered. The dose is preferably 650-750 mcg/m2/day, more preferably about 700 mcg/m2/day. The infusion time is preferably between 1 and 24 hours, more preferably between 1 and 3 hours. Especially preferred is an infusion time of about 1 or about 3 hours. The resting period is preferably 2-3 weeks, more preferably about 2 weeks.

The present invention also provides a compound of the invention for use in the treatment of a human patient afflicted with cancer, comprising administering to said patient a compound of the invention once weekly at a dose below 800 mcg/m2/day. The dose is preferably 600-700 mcg/m2/day, more preferable 650 mcg/m2/day. The infusion time is preferably between 1 and 24 hours, more preferably between 1 and 3 hours.

Although guidance for the dosage is given above, the correct dosage of the compound will vary according to the particular formulation, the mode of application, and the particular situs, host and tumour being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

The present invention is particularly directed to the compound of the present invention for use in the treatment of patients affected with prostate cancer, breast cancer, hepatocellular carcinoma, melanoma, colorectal cancer, renal cancer, ovarian cancer, NSCL cancer, epithelial cancer, pancreatic cancer and tumors that overexpress the Her2/neu oncogene. Most preferably it is directed to the compound of the present invention for use in the treatment of hepatocellular cancer, melanoma, breast cancer, pancreatic cancer and prostate cancer.

The present invention is also directed to the compound of the present invention for use in a method of treating a skin disease involving hyperproliferation of dermis cells in a mammal which comprises administering to the mammal an effective, non-toxic amount of a compound of the invention. The skin disease is preferably psoriasis. The present invention is preferably directed to the treatment of human patients affected with psoriasis, in particular severe psoriasis.

The compounds and compositions of this invention may be used with other drugs to provide a combination therapy. The other drugs may form part of the same composition, or be provided as a separate composition for administration at the same time or a different time. The identity of the other drug is not particularly limited, although combination with other chemotherapeutic, hormonal or antibody agents is envisaged. The amounts of the compound of the invention and the other pharmaceutically active agent or agents and the relative timings of administration will be selected in order to achieve the desired combined therapeutic effect.

### EXAMPLES

### General Procedures.

Cl-TrtCl-resin, Protected Fmoc-amino acid derivatives, HOBt, HOAt were from ABI (Framingham, MA), Bachem (Bubendorf, Switzerland), NovaBiochem (Läufelfingen, Switzerland), 4-MeHex derivatives from Narchem, HATU and other guanidylation derivatives were from ABI (Framingham, MA) or prepared as in del Fresno, M.; El-Faham, A.; Carpino, L.A.; Royo, M.; Albericio, F. Organic Lett., 2000, 2, 3539-3542.

Alloc-amino acids were prepared essentially as described by Dangles et al. see Dangles, O.;Guibé, F.; Balavoine, G.; Lavielle, S.; Marquet. A. J. Org. Chem. 1987, 52, 4984-4993 and Alloc-Z-Dhb-Phe-OH and Kahalalide F as described in WO 01 58934, DIPEA, DIPCDI, EDC·HCl, Piperidine, TFA were from Aldrich (Milwaukee, WI). DMF and CH₂Cl₂ were from SDS (Peypin, France). Acetonitrile (HPLC grade) was from Scharlau (Barcelona, Spain). All comercial reagents and solvents were used as received with exception of CH₂Cl₂, which was passed through a alumina column to remove acidic contaminants.

Solid-phase syntheses were carried out in polypropylene syringes (10-50 mL) fitted with a polyethylene porous disc. Solvents and soluble reagents were removed by suction. Removal of the Fmoc group was carried out with piperidine-DMF (2:8, v/v) (1 x 2 min, 2 x 10 min). Washings between deprotection, coupling, and, again, deprotection steps were carried out with DMF (5 x 0.5 min) and CH₂Cl₂ (5 x 0.5 min) using each time 10 mL solvent/g resin. Peptide synthesis transformations and washes were performed at 25 °C. Syntheses carried out on solid-phase were controlled by HPLC of the intermediates obtained after cleaving with TFA-H₂O (1:99) for 1 min an aliquot (aprox. 2 mg) of the peptidyl-resin. HPLC reversed phase columns [Nucleosil-C₁₈ 4,6 x 250 mm, 10 µm (column A); Nucleosil-C₄ 4,6 x 250 mm, 10 µm (column B) were from Sharlau (Spain); Symmetry™ C₁₈ 4,6 x 150 mm, 5 µm (column C); Symmetry300™ C₁₈ 4,6 x 50 mm, 5 µm (column D) were from Waters (Ireland) and Zorbax SB C₁₈ 4,6 x 150 mm, 3.5 µm (column E) was from Agilent (USA)]. Analytical HPLC was carried out on a Waters instrument comprising two solvent delivery pumps (Waters 1525), automatic injector (Waters 717 autosampler), dual wavelength detector (Waters 2487), and system controller (Breeze V3.20) and on a Agilent 1100 instrument comprising two solvent delivery pumps (G1311A), automatic injector (G1329A), DAD (G1315B). UV detection was at 215 or 220 nm, and linear gradients of CH₃CN (+0.036% TFA) into H₂O (+0.045% TFA) were run in the following conditions:

**Table I**

| **Chromatographic Conditions** | **Flow (mL/min)** | **Gradient (% of CH₃CN)** | **Runing Time (min)** |
|---|---|---|---|
| A | 1.0 | 30 to 100 | 30 |
| B | 1.0 | 40 to 60 | 15 |
| C | 1.0 | 45 to 60 | 8 |
| D | 1.0 | 40 to 70 | 8 |
| E | 1.0 | 45 to 70 | 8 |
| F | 1.0 | 40 to 70 | 15 |
| G | 1.0 | 40 to 65 | 15 |
| H | 1.0 | 10 to 100 | 30 |
| I | 1.0 | 45 to 65 | 15 |
| J | 1.0 | 40 to 70 | 15 |
| K | 1.0 | 55 to 75 | 15 |
| L | 1.0 | 40 to 100 | 15 |
| M | 1.0 | 50 to 100 | 8 |
| N | 1.0 | 35 to 60 | 15 |
| O | 1.0 | 50 to 100 | 30 |
| P | 1.0 | 50 to 100 | 15 |
| Q | 1.0 | Isocratic at 45 | 15 |
| R | 1.0 | 30 to 100 | 15 |
| S | 1.0 | 20 to 100 | 8 |
| T | 0.6 | 35 to 90 | 25 |
| U | 0.7 | 40 to 70 | 50 |
| V | 0.8 | 10 to 48 in 15 min and isocratic 30 min | 45 |
| W | 1.0 | 10 to 70 | 50 |
| X | 1.0 | 10 to 48 in 15 min and isocratic 30 min | 45 |
| Y | 0.8 | 10 to 60 | 50 |
| Z | 0.7 | 15 to 70 | 55 |
| AB | 0.8 | 10 to 60 | 55 |
| AC | 0.8 | 30 to 60 | 45 |
| AD | 0.8 | 10 to 48 in 10 min and isocratic 30 min | 40 |
| AE | 1.0 | 45 to 90 | 25 |
| AF | 1.0 | 40 to 100 | 8 |

MALDI-TOF and ES-MS analysis of peptide samples were performed in a PerSeptive Biosystems Voyager DE RP, using DHB matrix, and in a Waters Micromass ZQ spectrometer and in an Agilent Ion Trap 1100 Series LC/MSDTrap. Peptide-resin samples were hydrolyzed in 12 N aqueous HCl-propionic acid (1:1), at 155 °C for 1-3 h and peptide-free samples were hydrolyzed in 6 N aqueous HCl at 155 °C for 1 h. Subsequent amino acid analyses were performed on a Beckman System 6300 autoanalyzer. ¹H-NMR spectroscopy [1H, NOESY, TOCSY at (278K)] was performed on a Varian Unity Plus (500 MHz). Chemical shifts (δ) are expressed in parts per million downfield from TMS. Coupling constants are expressed in hertz.

The names of the analogues are named relative to the 5-methyhexyl isomer of Kahalalide F of formula (I), indicating between square brackets the modified residue; the suffix "no" indicates the elimination of the natural residue from the sequence.

### Example 1

### (4S)-MeHex-D-Val-ThrVal-D-Val-D-Pro-Orn-D-allo-Ile-cyclo[D-allo-Thr-D-allo-Ile-D-Val-Phe-(Z)Dhb-Val], [(4S)-MeHex¹⁴]-Kahalalide F

### (Reference Compound 1)

### Step 1

### H-D-Val-O-TrtCl-resin.

Cl-TrtCl-resin (1 g, 1.64 mmol/g) was placed in a 20 mL polypropylene syringe fitted with a polyethylene filter disk. The resin was then washed with CH₂Cl₂ (5 x 0.5 min), and a solution of Fmoc-D-Val-OH (238 mg, 0.7 mmol, 0.7 equiv) and DIPEA (0.41 mL) in CH₂Cl₂ (2.5 mL) was added, and the mixture was stirred for 15 min, when extra DIPEA (0.81 mL, total 7 equiv, 7 mmol) and the mixture stirred for 45 min. The reaction was terminated by addition of MeOH (800 µL), after a stirring of 10 min. The Fmoc-D-Val-O-TrtCl-resin was subjected to the following washings/treatments with CH₂Cl₂ (3 x 0.5 min), DMF (3 x 0.5 min), piperidine as indicated in General Procedures, and DMF (5 x 0.5 min). The loading calculated by Fmoc determination was 0.50 mmol/g.

### Step 2

### Fmoc-D-allo-Ile-D-allo-Thr(Val-Alloc)-D-allo-Ile-D-Val-O-TrtCl-resin.

Fmoc-D-*allo*-Ile-OH (707 mg, 2 mmol, 4 equiv), Fmoc-D-*allo*-Thr-OH (free hydroxy group) (683 mg, 2 mmol, 4 equiv), and Fmoc-D-*allo-*Ile-OH (707 mg, 2 mmol, 4 equiv) were added sequentially to the above obtained H-D-Val-O-TrtCl-resin using DIPCDI (310 µL, 2 mmol, 4 equiv) and HOBt (307 mg, 2 mmol, 4 equiv) in DMF (2.5 mL). In all cases, after 90 min of coupling, the ninhydrin test was negative. Removal of Fmoc group and washings were carried out as described in General Procedures. Alloc-Val-OH (502 mg, 2.5 mmol, 5 equiv) was coupled with DIPCDI (387 mg, 2.5 mmol, 5 equiv) in the presence of DMAP (30.6 mg, 0.25 mmol, 0.5 equiv) and DIPEA (88 µL, 0.5 mmol, 1 equiv) for 45 min. This coupling was repeated in the same conditions twice. An aliqout of the peptidyl-resin was treated with TFA and the HPLC (t*_{R}* 7.8 min, conditions S, column D) of the crude obtained after evaporation showed a purity of > 98%. ESMS, calcd for C₄₅H₆₃N₅O₁₁, 849.45.
Found: *m*/*z* 850.1 [M+H]⁺.

### Step 3

### Fmoc-D-Val-D-Pro-Orn(Boc)-D-allo-Ile-D-allo-Thr(Val-Alloc)-D-allo-Ile-D-Val-O-TrtCl-resin.

The Fmoc group was removed and Fmoc-Orn(Boc)-OH (912 mg, 2 mmol, 4 equiv), Fmoc-D-Pro-OH (843 mg, 2.5 mmol, 5 equiv), and Fmoc-D-Val-OH (255 mg, 2.5 mmol, 5 equiv) were sequentially added to the above peptidyl-resin (Step 2) using DIPCDI (310 µL, for 2.0 mmol and 4 equiv; and 388 µL, for 2.5 mmol, 5 equiv) and HOBt (307 mg, for 2.0 mmol and 4 equiv; and 395 mg, 2.5 mmol. 5 equiv) for 90 min. Ninhydrin test after incorporation of Orn and D-Pro was negative. The chloranil after incorporation of D-Val was slightly positive and therefore a recoupling of this residue was carried out with Fmoc-D-Val-OH (678 mg, 2.0 mmol, 4 equiv), DIPCDI (310 µL, 2.0 mmol, 4 equiv) and HOBt (307 mg, 2.0 mmol, 4 equiv) for 90 min. An aliquot of the peptidyl-resin was treated with TFA and the HPLC (t*_{R}* 10.1 min, conditions S, column D) of the crude obtained after evaporation showed a purity of > 98 %. MALDI-TOF-MS, calcd for C₆₅ H₉₇ N₉ O₁₆, 1,259.71. Found: *m*/*z* 1,282.16 [M+Na]⁺.

### Step 4

### (4S)-MeHex-D-Val-Thr(tBu)-Val-D-Val-D-Pro-Orn(Boc)-D-allo-Ile-D- allo-Thr(Val-Alloc)-D-allo-Ile-D-Val-O-TrtCl-resin

The Fmoc group was removed and Fmoc-Val-OH (678 mg, 2 mmol, 4 equiv), Fmoc-Thr(*t*Bu)-OH (992 mg, 2.5 mmol, 5 equiv), Fmoc-D-Val-OH (678 mg, 2 mmol, 4 equiv), and (4S)-MeHex-OH (195 mg, 1.5 mmol, 3 equiv) were sequentially added to the above peptidyl-resin (Step 3) using DIPCDI (233 µL, for 1.5 mmol and 3 equiv; 310 µL, for 2 mmol and 4 equiv; and 388 µL, for 2.5 mmol, 5 equiv) and HOBt (230 mg, for 1.5 mmol and 3 equiv; 307 mg, for 2 mmol and 4 equiv; and 395 mg, 2.5 mmol. 5 equiv) for 90 min: In all cases, after 90 min of coupling, the ninhydrin test was negative. Removal of Fmoc group and washings were carried out as described in General Procedures.

### Step 5

### (4S)-MeHex-D-Val-Thr(tBu)-Val-D-Val-D-Pro-Orn(Boc)-D-allo-Ile-D-allo-Thr(Val-Z-Dhb-Phe-Alloc)-D-allo-Ile-D-Val-O-TrtCl-resin.

Alloc group was removed with Pd(PPh₃)₄ (58 mg, 0.05 mmol, 0.1 equiv) in the presence of PhSiH₃ (617 µL, 5 mmol, 10 equiv) under atmosphere of Ar and Alloc-Phe-Z-Dhb-OH (666 mg, 2 mmol, 4 equiv) and HOAt (273 mg, 2 mmol, 4 equiv) were dissolved in DMF (1.25 mL) and added to peptidyl-resin, then DIPCDI (310 µL, 2 mmol, 4 equiv) was added and the mixture stirred for 5 h, where the ninhydrin test was negative. After washings with DMF and CH₂Cl₂, an aliquot of the peptidyl-resin was treated with TFA-H₂O (1:99) for 1 min and the product was characterized by MALDI-TOF-MS, calcd for C₈₈ H₁₄₆ N₁₄ O₂₁, 1,735.08. Found: *m*/*z* 1,758.67 [M+Na]⁺, 1,774.62 [M+K]⁺.

### Step 6

### (4S)-MeHex-D-Val-Thr(tBu)-Val-D-Val-D-Pro-Orn(Boc)-D-allo-Ile-D-allo-Thr(Val-Z-Dhb-Phe-H)-D-allo-Ile-D-Val-OH.

After washings with DMF and CH₂Cl₂, the Alloc group was removed with Pd(PPh₃)₄ (58 mg, 0.05 mmol, 0.1 equiv) in the presence of PhSiH₃ (617 µL, 5 mmol, 10 equiv) under atmosphere of Ar. The protected peptide was cleaved from the resin by TFA-CH₂Cl₂ (1:99) (5 x 30 sec). Filtrate was collected on H₂O (4 mL) and the H₂O was partially removed in a rotavapor. ACN was then added to dissolve solid that appeared during the H₂O removal, and the solution was lyophilized, to give 639 mg (387 µmol, 77% yield) of the title compound with a purity of > 95 % as checked by HPLC (Condition R, column C, t*_{R}* 10.5 min).

### Step 7

### (4S)-MeHex-D-Val-Thr-Val-D-Val-D-Pro-Orn-D-allo-Ile-cyclo(D-allo-Thr-D-allo-Ile-D-Val-Phe-Z-Dhb-Val)

The protected peptide (Step 6) (639 mg, 387 µmol) was dissolved in CH₂Cl₂ (390 mL, 1 mM), and HOBt (237 mg, 1.55 mmol) dissolved in the minimum volume of DMF to dissolve HOBt, DIPEA (203 µL, 1.16 mmol, 3 equiv), and DIPCDI (240 µL, 1.55 mmol, 4 equiv) were added. The mixture was allowed to stir for 1 h, then the course of the cyclization step was checked by HPLC. The solvent was removed by evaporation under reduced pressure. The protected cyclic peptide was dissolved in TFA-H₂O (19:1, 85 mL) and the mixture was allowed to stir for 1 h. The solvent was removed by evaporation under reduced pressure, and dioxane is added (30 mL) and the solvent is removed by evaporation under reduced pressure (the process was repeated three times), then H₂O (40 mL) was added and lyophilized. The crude product was purified by HPLC (Kromasil C₈ 5 µm, 205 x 50 mm), isocratic 44% acetonitrile (+0.05% TFA) in water (+0.05% TFA), 55 mL/h, detection at 220 nm, to give the title product (192 mg, 0.13 mmol, 26% yield, 92.3%). MALDI-TOF-MS, calcd for C₇₅ H₁₂₄ N₁₄ O₁₆, 1,476.93. Found: *m*/*z* 1,500.12 [M+Na]⁺, 1,515.97 [M+K]⁺. The ¹H-NMR (2.5 mM, 500 MHz, H₂O-D₂O (9: 1) spectrum of the compound is indicated in Table II).

**Table II**

| RESIDUE | N-H | Hα | Hβ | OTHER |
|---|---|---|---|---|
| (Z)-Dhb | 9.59 (s) | - | 6.63 (q, J =7.5 Hz) | 1.19 (d, γ-CH₃) |
| D-al·lo-Ile 1 | 8.82 (d, J =9.0 Hz) | 4.42 | 1.87 | 1.25, 1.09, 0.82 (γ-CH₂, γ-CH₃, δ-CH₃) |
| L-Phe | 8.75 (d, J =5.5 Hz) | 4.63 | 3.08 (m) | 7.31 (2H Ar, t), 7.25 (3H Ar, d) |
| D-al·lo-Thr | 8.67(d, J=9.0 Hz) | 4.64 | 5.05 (m) | 1.21 (γ-CH₃) |
| D-Val 3 | 8.13 (d, J =7.5 Hz) | 4.33 | 2.01 | 0.90 (2 γ-CH₃) |
| L-Orn | 8.29 (d, J =7.5 Hz) | 4.31 | 1.66 (2H) | 1.88 (γ-CH₂), 2.96 (bs, δ-CH₂), 7.56 (ε-NH₃⁺) |
| D-al·lo-Ile 2 | 7.92 (d) | 4.18 | 1.80 | 1.25, 1.09, 0.81 (γ-CH₂, γ-CH₃, δ-CH₃) |
| D-Val 5 | 8.01 (d) | 4.08 | 2.07 | 0.87 (2 γ-CH₃) |
| L-Thr | 8.19 (d, J =7.5 Hz) | 4.29 | 4.14 (m) | 1.13 ( γ-CH₃) |
| D-Val 2 | 7.89 (d, J =7.5 Hz) | 4.32 | 2.11 | 0.78 (γ-CH₃) |
| L-Val 4 | 8.04 (d) | 4.10 | 2.07 | 0.90 (2 γ-CH₃) |
| L-Val 1 | 7.19 (d, J =9.0 Hz) | 4.02 | 1.52 | 0.75 (γ-CH₃), 0.65 (d, γ-CH3) |
| D-Pro | - | 4.36 | 2.23, 1.99 (m, β-CH₂), 1.85 (m, γ-CH₂), 3.83 (1H, m, δ-CH₂), 3.64 (1H, m, δ-CH₂) | |
| 4(S)-MeHex | - | 2.26 (2H) | 1.57 (β-CH₂), 1.26, 1.10, 1.33, 0.79 (δ-CH₂, δ-CH₃, γ-CH, ε-CH₃) | |

### Example 2

The analogue of the present invention described in Table III was synthesized following experimental procedures as described in Example 1, except the step indicated into the column (Step), where residue(s) (A) was replaced by other(s) (B) or removed (none).

**Table III**

| **Analogue** | **Compound #** | **Step** | **Residue Replaced (A)** | **Residue Incorporated (B)** |
|---|---|---|---|---|
| [Orn¹¹]-KF | 49 | 4 | Fmoc-Val-OH 11 | Fmoc-Orn-OH |
| | | | (4S)-MeHex 14 | 5-MeHex |

### Characterization of the Kahalalide F analogues

Characterization is showed in Table VI and VII. EM in Table VI corresponds to MALDI-TOF and the exact mass was calculated by Chemwind 6.0, and EM in Table VII corresponds to APCI in positive mode.

**Table VI**

| **Analogue** | **Compound #** | **Chromatography** | | **t_{R} (min)** | **Exact mass** | **Found** |
|---|---|---|---|---|---|---|
| | | Cond. | Colum | | | |
| [(4S)-MeHex¹⁴]-KF | Reference Compound 1 | N | C | 10.22 | 1,476.93 | 1,500.1/1,516.0 |
| [Orn¹¹]-KF | 49 | Y | E | 35.70 | 1,491.94 | 1,493.4/1,515.1/74 |
| | | | | | | 7.5 |

### Example 17

### Biological activity

The bioactivity of compounds of this invention is demonstrated by the results in the following tables obtained in accordance with the methodology described as follows:
The finality of this assay is to interrupt the growth of a *"in vitro"* tumor cell culture by means a continued exhibition of the cells to the sample to be testing.

### Cell Lines

| **NAME** | **N° ATCC** | **SPECIES** | **TISSUE** | **CHARACTERISTICS** |
|---|---|---|---|---|
| A-549 | CCL-185 | human | lung | lung carcinoma "NSCL" |
| SK-MEL-28 | HTB-72 | human | melanoma | malignant melanoma |
| HT-29 | HTB-38 | human | colon | colon adenocarcinoma |
| LoVo | CCL-229 | human | colon | colon adenocarcinoma |
| LoVo-Dox | | human | colon | colon adenocarcinoma (MDR) |
| DU-145 | HTB-81 | human | prostate | prostate carcinoma, not androgen receptors |
| LNCaP | CRL-1740 | human | prostate | prostate adenocarcinoma, with androgen receptors |
| SK-BR-3 | HTB-30 | human | breast | breast adenocarcinoma, Her2/neu+, (pleural effusion) |
| IGROV-1 | | human | ovary | ovary adenocarcinoma |
| IGROV-ET | | human | ovary | ovary adenocarcinoma, characterized as ET-743 resistant cells |
| SK-OV-3 | HTB-77 | human | ovary | ovary adenocarcinoma (malignant ascites) |
| HeLa | CCL-2 | human | cervix | cervix epitheloid carcinoma |
| HeLa-APL | CCL-3 | human | cervix | cervix epitheloid carcinoma, characterized as aplidine resistant cells |
| K-562 | CCL-243 | human | bone marrow; | chronic myelogenous leukemia |
| PANC-1 | CRL-1469 | human | pancreas | pancreatic epitheloid carcinoma |
| HMEC-1 | | human | endothelium | |

A colorimetric type of assay, using sulforhodamine B (SRB) reaction has been adapted for a quantitative measurement of cell growth and viability [following the technique described by Philip Skehan, et al. (1990), New colorimetric cytotoxicity assay for anticancer drug screening, J. Natl. Cancer Inst., 82:1107-1112].

This form of the assay employs 96 well cell culture microplates of 9 mm diameter (Faircloth, 1988; Mosmann, 1983). Most of the cell lines are obtained from American Type Culture Collection (ATCC) derived from different human cancer types.

Cells are maintained in RPMI 1640 10% FBS, supplemented with 0.1 g/l penicillin and 0.1 g/l streptomycin sulfate and then incubated at 37°C, 5% CO₂ and 98% humidity. For the experiments, cells were harvested from subconfluent cultures using trypsin and resuspended in fresh medium before plating.

Cells are seeded in 96 well microtiter plates, at 5 x 10³ cells per well in aliquots of 195 µL medium, and they are allowed to attach to the plate surface by growing in drug free medium for 18 hours. Afterward, samples are added in aliquots of 5 µL in a ranging from 10 to 10⁻⁸ µg/mL, dissolved in DMSO:EtOH:PBS (0.5:0.5:99). After 48 hours exposure, the antitumor effect are measured by the SRB methodology: cells are fixed by adding 50 µL of cold 50% (wt/vol) trichloroacetic acid (TCA) and incubating for 60 minutes at 4°C. Plates are washed with deionized water and dried. One hundred µL of SRB solution (0.4% wt/vol in 1% acetic acid) is added to each microtiter well and incubated for 10 minutes at room temperature. Unbound SRB is removed by washing with 1% acetic acid. Plates are air dried and bound stain is solubilized with Tris buffer. Optical densities are read on a automated spectrophotometric plate reader at a single wavelength of 490 nm.

The values for mean +/- SD of data from triplicate wells are calculated. Some parameters for cellular responses can be calculated: GI = growth inhibition, TGI = total growth inhibition (cytostatic effect) and LC = cell killing (cytotoxic effect).

Tables VIII and IX illustrate data on the biological activity of the compounds of the present invention. Compound 1 refers to Reference Compound 1.

**Table VIII: Activity data (Molar)**

| | | **DU-145** | **LN-caP** | **SKO V-3** | **IGROV** | **IGROV-ET** | **SK-BR-3** | **MEL-28** | **H-M EC-1** |
|---|---|---|---|---|---|---|---|---|---|
| **1** | GI50 | 3,27E-07 | 9,00E-07 | - | 1,58E-07 | 1,35E-07 | 4,34E-08 | 3,15E-07 | - |
| | TGI | 8,80E-07 | 2,14E-06 | - | 3,19E-07 | 2,89E-07 | 9,27E-08 | 4,10E-07 | - |
| | LC50 | 2,54E-06 | 5,08E-06 | - | 6,41E-07 | 6,19E-07 | 4,69E-07 | 2,10E-06 | - |
| | | **DU-145** | **LN-caP** | | **IGROV** | **IGROV-ET** | **SK-BR-3** | **MEL-28** | |
| **49** | GI50 | 1,09E-06 | 2,20E-06 | | 1,27E-06 | 1,91E-06 | 1,94E-06 | | 1,33E-06 |
| | TGI | 2,50E-06 | 6,70E-06 | | 2,75E-06 | 5,15E-06 | 5,57E-06 | | 2,50E-06 |
| | LC50 | 5,75E-06 | 6,70E-06 | | 5,97E-06 | 6,70E-06 | 6,70E-06 | | 4,69E-06 |

| | | **A-549** | **K-562** | **PANC-1** | **HT-29** | **LOVO** | **LOVO-DOX** | **HELA** | **HELA-APL** |
|---|---|---|---|---|---|---|---|---|---|
| **49** | GI50 | 2,61E-06 | 1,50E- 07 | 1,31E-06 | 1,58E-06 | 1,47E-06 | 3,22E-06 | 1,10E-06 | 1,07E-06 |
| | TGI | 6,70E-06 | 1,67E-06 | 3,09E-06 | 3,74E-06 | 3,80E-06 | 6,70E-06 | 2,20E-06 | 2,52E-06 |
| | LC50 | 6,70E-06 | 6,70E-06 | 6,70E-06 | 6,70E-06 | 6,70E-06 | 6,70E-06 | 4,40E-06 | 5,95E-06 |

## Claims

1. A compound based on the structure of kahalalide F of formula 1, designated KF 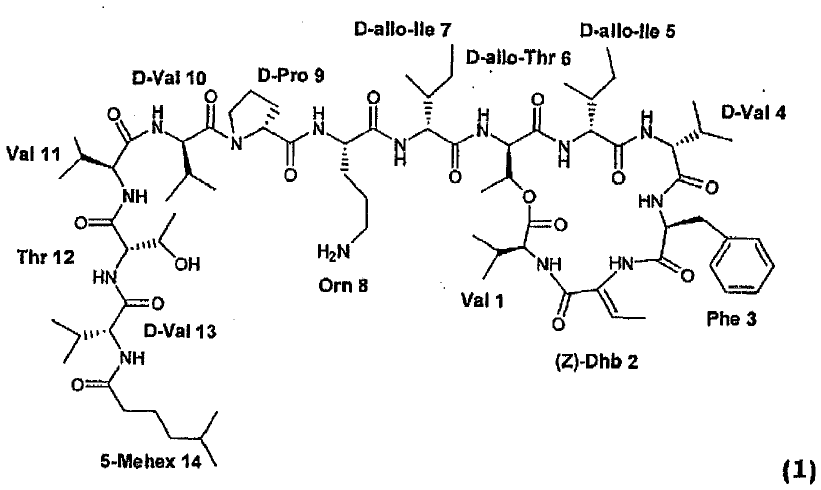 which is [Orn¹¹]-KF, wherein the amino acid indicated between brackets is the modification introduced in the structure of kahalalide F;
or a pharmaceutical acceptable salt thereof.

2. A pharmaceutical composition comprising a compound according to claim 1 and a pharmaceutically acceptable carrier, vehicle or diluent.

3. A compound or pharmaceutical composition according to claim 1 or claim 2 for use as a medicament.

4. A compound or a pharmaceutical composition according to any of claims 1 to 3 for use in the treatment of cancer.

## Patentansprüche

1. Verbindung, die auf der Struktur von Kahalalide F der Formel 1 basiert, bezeichnet als KF: 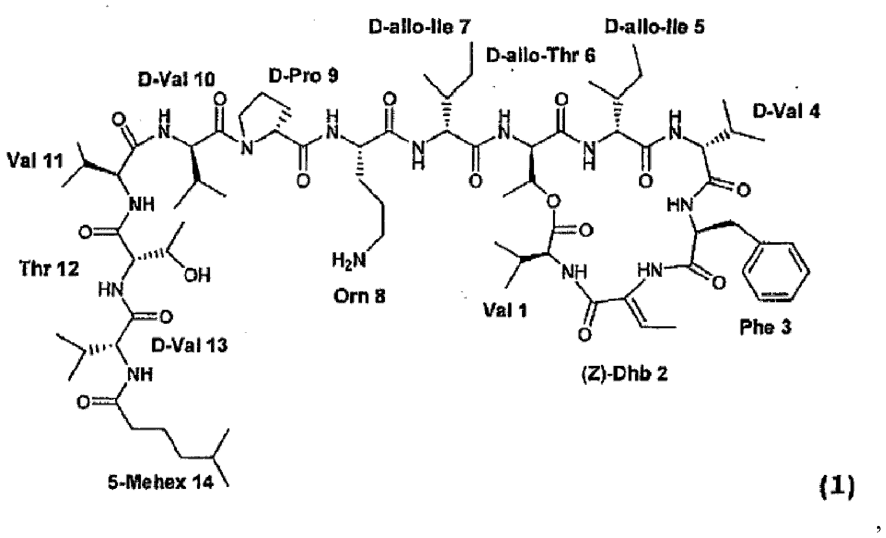 die [Orn¹¹]-KF ist, worin die Aminosäure, die zwischen Klammern angegeben wird, die in die Struktur von Kahalalide F eingeführte Modifikation ist;
oder ein pharmazeutisch akzeptables Salz davon.

2. Pharmazeutische Zusammensetzung, die eine Verbindung nach Anspruch 1 und eine pharmazeutisch akzeptable Trägersubstanz, ein Trägermedium oder Verdünnungsmittel aufweist.

3. Verbindung oder pharmazeutische Zusammensetzung nach Anspruch 1 oder Anspruch 2 für eine Verwendung als ein Medikament.

4. Verbindung oder pharmazeutische Zusammensetzung nach einem der Ansprüche 1 bis 3 für eine Verwendung bei der Behandlung von Krebs.

## Revendications

1. Composé basé sur la structure du kahalalide F de la formule 1, désigné KF: 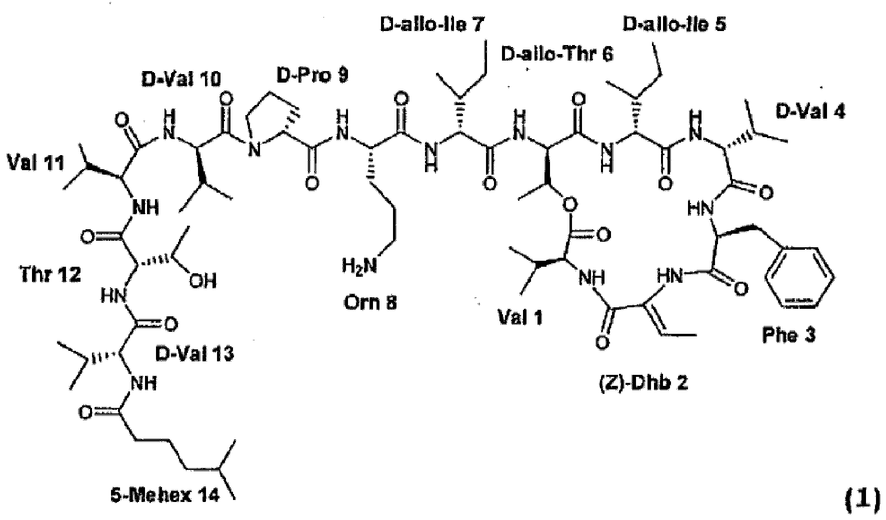 qui est [Orn¹¹]-KF, dans lequel l'acide aminé indiqué entre crochets est la modification introduite dans la structure du kahalalide F ;
ou un sel pharmaceutiquement acceptable de celui-ci.

2. Composition pharmaceutique comprenant un composé selon la revendication 1 et un support, un véhicule ou un diluant pharmaceutiquement acceptable.

3. Composé ou composition pharmaceutique selon la revendication 1 ou 2 pour une utilisation en tant que médicament.

4. Composé ou composition pharmaceutique selon l'une quelconque des revendications 1 à 3 pour une utilisation dans le traitement d'un cancer.
